(19) Europäisches Patentamt
European Patent Office
Office européen des brevets

(11) Veröffentlichungsnummer : **0 029 175**
**B1**

(12) # EUROPÄISCHE PATENTSCHRIFT

(45) Veröffentlichungstag der Patentschrift : **15.12.82**

(51) Int. Cl.[3] : **C 07 C121/80**, C 07 C120/00, C 07 B 19/00

(21) Anmeldenummer : **80106809.9**

(22) Anmeldetag : **05.11.80**

(54) **Verfahren zur Gewinnung der enantiomeren Formen von 4-Cyan-1-(N-methyl-N-2'-(3'',4''-dimethoxyphenyl)-ethyl)-amino)-5-methyl-4-(3',4',5'-trimethoxyphenyl)-hexan und dessen Salzen.**

(30) Priorität : **17.11.79 DE 2946545**

(43) Veröffentlichungstag der Anmeldung : **27.05.81 (Patentblatt 81/21)**

(45) Bekanntmachung des Hinweises auf die Patenterteilung : **15.12.82 Patentblatt 82/50**

(84) Benannte Vertragsstaaten : **AT BE CH DE FR GB IT LI NL**

(56) Entgegenhaltungen :
**DE A 2 813 712**
**DE B 1 593 921**

(73) Patentinhaber : **BASF Aktiengesellschaft**
**Carl-Bosch-Strasse 38**
**D-6700 Ludwigshafen (DE)**

(72) Erfinder : **Herrling, Siegfried, Dr.**
**Dohlenweg 33**
**D-5190 Stolberg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 029 175 B1

Verfahren zur Gewinnung der enantiomeren Formen von 4-Cyan-1-(N-methyl-N-(2'-(3'',4''-dimethoxyphenyl)-ethyl)-amino)-5-methyl-4-(3',4',5'-trimethoxyphenyl)-hexan und dessen Salzen

Die deutschen Patentschriften 1 154 810 und 1 158 083 beschreiben die Herstellung bestimmter basisch substituierter Phenylacetonitrile, die z.B. der folgenden Formel entsprechen

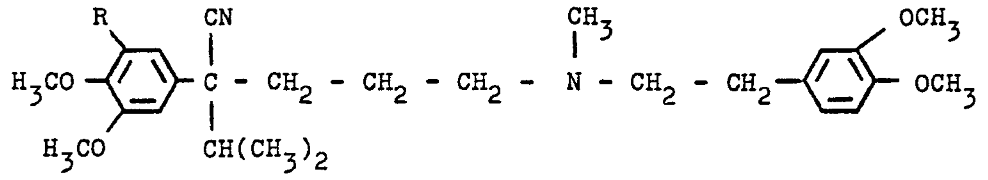

(I)

worin R Wasserstoff oder eine Methoxygruppe bedeutet, in razemischer Form. Die Gewinnung der optisch aktiven Formen durch Aufspaltung des Razemates gelang für die Verbindung mit R = Wasserstoff nicht (Helv. chim. Acta, *58* (1975) 2 050 ff), so daß eine vielstufige Synthese, beginnend mit optisch aktiven Ausgangsmaterialien, entwickelt wurde, um die Enantiomeren dieser Verbindung zu erhalten.

Die DE-PS 2 059 923 beschreibt u.a. die Herstellung der 1-Form der Verbindung der Formel I, in der R = Wasserstoff ist, nach mehrstufigen Verfahren, und zwar ausschließlich unter Verwendung optisch aktiver Ausgangsmaterialien.

Die deutsche Patentschrift 2 059 985 betrifft u.a. bestimmte, rechtsdrehende, basisch substituierte Phenylacetonitrile und Verfahren zu deren Herstellung, wobei einige der Produkte unter die obige Formel I fallen. Auch in dieser Literaturstelle werden — wie in den vorhergehenden Zitaten — zur Herstellung der optisch aktiven Verbindungen ausschließlich mehrstufige Synthesen unter Verwendung von optisch aktiven Ausgangsmaterialien beschrieben.

Nach diesem Stand der Technik mußte man also davon ausgehen, daß eine Razemattrennung bei in bestimmter Weise substituierten Phenylacetonitrilen, insbesondere bei den Verbindungen der Formel I, nicht möglich sei.

Überraschenderweise wurde nun gefunden, daß man die Enantiomeren des 4-Cyan-1-[N-methyl-N-(2'-(3'',4''-dimethoxyphenyl)-ethyl)-amino]-5-methyl-4-(3',4',5'-trimethoxyphenyl)-hexans (I, R = $CH_3O$, im folgenden als Verbindung Ia bezeichnet) bzw. deren Salze in guter Ausbeute aus dem Razemat dieser Verbindung Ia erhalten kann, indem man zunächst das saure Salz aus dieser Verbindung und einem Mol einer optisch aktiven Form, vorzugsweise der D-Form, der O,O'-Dibenzoylweinsäure herstellt, durch Kristallisation die diastereomeren Formen dieses Salzes trennt und dann daraus in üblicher Weise die freie Base der Verbindung Ia in enantiomerer Form oder ein anderes Salz, wie z.B. das Hydrochlorid gewinnt.

Die Herstellung des sauren Salzes der optisch aktiven Form der O,O'-Dibenzoylweinsäure mit der Verbindung Ia erfolgt durch doppelte Umsetzung (beispielsweise Umsetzung des Mono-Natriumsalzes der (+)-O,O'-Dibenzoyl-(D)-weinsäure mit dem Hydrochlorid der Verbindung Ia) oder auch durch Neutralisation der freien Base mit freier (+)-O,O'-Dibenzoyl-(D)-weinsäure in Gegenwart eines geeigneten Lösungsmittels, wie z.B. eines niederen Alkohols, insbesondere Isopropanol.

Die unterschiedlichen Stärken in der pharmakologischen Wirkung der beiden Enantiomeren sind beispielsweise Brit. J. Pharmacol. *59*, 411 (1977) zu entnehmen.

Die folgenden Beispiele erläutern die Erfindung näher. Alle Temperaturangaben darin sind unkorrigiert. Bei der Durchführung dieser Beispiele wurde auf die Erzielung optimaler Ausbeuten kein Wert gelegt.

## Beispiel 1

a) 26 g d,l-4-Cyan-1-[N-methyl-N-(2'-(3'',4''-dimethoxyphenyl)-ethyl)-amino]-5-methyl-4-(3',4',5'-trimethoxyphenyl)-hexanhydrochlorid (Ia) werden in 500 ml 50 %igem Ethanol (Gemisch gleicher Teile Ethanol und Wasser) gelöst und unter Rühren mit einer Lösung von 18,8 g (+)-O,O'-Dibenzoyl-D-weinsäurehydrat und 2 g Natriumhydroxid in 50 ml 50 %igem Ethanol versetzt. Das Gemisch wird für 1 1/2 Stunden bei Raumtemperatur gerührt und dann für 15 Stunden im Kühlschrank aufbewahrt. Die ausgefallenen Kristalle werden abgesaugt, getrocknet und dann zweimal aus Isopropanol oder aus 60 %igem Ethanol umkristallisiert. Man erhält so das saure (+)-O,O'-Dibenzoyl-D-tartrat der d-Form der Verbindung Ia in einer Ausbeute von 17,4 g = 83 % der Theorie, $[\alpha]_D^{22}$ + 69,2° (10,6 mg/ml Ethanol), Schmelzpunkt : 123 °C. Diese Werte ändern sich bei weiterem Umkristallisieren nicht.

b) 17 g des in Beispiel 1a erhaltenen Salzes werden mit 100 ml Toluol und 80 ml 1N-Natronlauge kräftig gerührt oder geschüttelt, bis das Salz zerlegt ist und das Gemisch beim Stillstand zwei klare Schichten bildet. Diese Schichten werden getrennt, die Toluolschicht wird mit Wasser gewaschen und dann mit einem Gemisch aus 50 ml 1N-Salzsäure und 25 ml Wasser versetzt. Man saugt den Niederschlag ab und löst ihn unter gelindem Erwärmen in der von der Toluolschicht abgetrennten salzsauren Schicht.

Diese Lösung wird nach Filtrieren für einige Stunden unter Kühlung aufbewahrt. Der Niederschlag wird abgesaugt, mit etwas Eiswasser gewaschen und dann mit Toluol azeotrop getrocknet. Durch mehrfaches Umkristallisieren aus Isopropanol erhält man das d-4-Cyan-1-[N-methyl-N(2'-(3",4"-dimethoxyphenyl)-ethyl)-amino]-5-methyl-4-(3',4',5'-trimethoxyphenyl)-hexanhydrochlorid als weißes Kristallpulver in einer Ausbeute von 41,6 % der Theorie (bezogen auf die eingesetzte Menge des Produktes aus Beispiel 1a), $[\alpha]_D^{22}$ + 13,3° (12 mg/ml Ethanol), Schmelzpunkt : 159-161 °C.

Beispiel 2

a) Die in Beispiel 1a erhaltene Ethanol-haltige Mutterlauge wird im Vacuum auf ein kleines Volumen eingeengt und dann gekühlt. Die ausgefallenen Kristalle werden abgesaugt, mit Wasser gewaschen und dann aus Essigsäureethylester umkristallisiert, wobei man das saure (+)-O,O'-Dibenzoyl-D-tartrat der l-Form der Verbindung Ia erhält. Ausbeute : 10,2 g = 48,5 % der Theorie, $[\alpha]_D^{22}$ + 47,9° (9,82 mg/ml Ethanol), Schmelzpunkt : 87-90 °C.

b) 10 g des in Beispiel 2a erhaltenen Salzes werden entsprechend dem in Beispiel 1b beschriebenen Vorgehen behandelt, wobei man das l-4-Cyan-1-[N-methyl-N(2'-(3",4"-dimethoxyphenyl)-ethyl)-amino]-5-methyl-4-(3',4',5'-trimethoxyphenyl)-hexan-hydrochlorid als weißes Kristallpulver in einer Ausbeute von 2,8 g (= 45,3 % der Theorie) erhält, $[\alpha]_D^{20}$-13,4° (10,6 mg/ml Ethanol), Schmelzpunkt 158-161 °C.

Beispiel 3

a) Man löst 24,23 g d,l-4-Cyan-1-[N-methyl-N-(2'-(3",4"-dimethoxyphenyl)-ethyl)-amino]-5-methyl-4-(3',4',5'-trimethoxyphenyl)-hexan (Ia) in 75 ml Isopropanol und gibt dazu unter Rühren eine Lösung von 18,82 g (+)-O,O'-Dibenzoyl-D-weinsäurehydrat in 50 ml Isopropanol. Das Gemisch wird im Kühlschrank über Nacht aufbewahrt. Die Kristalle werden abgesaugt und dann zweimal aus Isopropanol umkristallisiert. Man erhält das gleiche Produkt wie in Beispiel 1a in einer Ausbeute von 18,1 g = 86 % der Theorie. Dieses kann wie in Beispiel 1b beschrieben weiter umgesetzt werden.

b) Die erste in Beispiel 3a erhaltene Mutterlauge wird im Vacuum eingedampft. Der Rückstand liefert nach Umkristallisieren aus Essigester das gleiche Produkt wie in Beispiel 2a beschrieben. Ausbeute : 13,7 g = 65 % der Theorie.

Beispiel 4

Man verfährt wie in den vorhergehenden Beispielen, verwendet aber statt des Hydrates der (+)-O,O'-Dibenzoyl-L-weinsäure 18,82 g (−)-O,O'-Dibenzoyl-L-weinsäure-hydrat und erhält so ebenfalls die d- bzw. die l-Form des Hydrochlorides des 4-Cyan-1-[N-methyl-N-(2'-(3",4"-dimethoxyphenyl)-ethyl)-amino]-5-methyl-4-(3',4',5'-trimethoxyphenyl)-hexans.

Beispiel 5

Man verfährt wie in Beispiel 1b, versetzt jedoch die mit Wasser gewaschene, das Produkt enthaltende Toluolschicht nicht mit wässriger Salzsäure, sondern mit einer Lösung von ca. 0,9 g Chlorwasserstoff in feuchtem Ether. Der Niederschlag wird abgesaugt und in ca. 40-50 °C warmer 0,5 N-Salzaüre gelöst. Diese salzsaure Lösung wird für einige Stunden im Kühlschrank aufbewahrt. Der Niederschlag wird wie in Beispiel 1b isoliert und weiterbehandelt, wobei man das gewünschte Produkt in einer Ausbeute von 48,5 % der Theorie (bezogen auf die eingesetzte Menge des Produktes aus Beispiel 1a) erhält.

**Ansprüche**

1. Verfahren zur Gewinnung der enantiomeren Formen von 4-Cyan-1-[N-methyl-N-(2'-(3",4"-dimethoxyphenyl)-ethyl)-amino]-5-methyl-4-(3',4',5'-trimethoxyphenyl)-hexan und dessen Salzen, dadurch gekennzeichnet, daß man durch Neutralisation der Freien Base oder durch doppelte Umsetzung eines Salzes dieser Verbindung deren saures Salz mit einer optisch aktiven Form der O,O'-Dibenzoylweinsäure als Diastereomerengemisch herstellt, dieses durch Kristallisation trennt und die so erhaltenen reinen Diastereomeren in an sich bekannter Weise in die freien Basen der enantiomeren Formen des 4-Cyan-1-[N-methyl-N-(2'-(3",4"-dimethoxyphenyl)-ethyl)-amino]-5-methyl-4-(3',4',5'-trimethoxyphenyl)-hexans oder in deren Salze überführt.

2. Verfahren gemäß Anspruch 1, dadurch gekennzeichnet, daß das saure O,O'-Dibenzoyltartrat als Diastereomerengemisch unter Verwendung von Isopropanol als Lösungsmittel hergestellt wird.

3. Verfahren gemäß Ansprüchen 1 und 2, dadurch gekennzeichnet, daß die (+)-O,O'-Dibenzoyl-D-weinsäure, ihr Hydrat oder eines ihrer Monoalkalisalze Anwendung finden.

**Claims**

1. A process for obtaining the enantiomeric forms of 4-cyano-1-[N-methyl-N-(2′-(3″,4″-dimethoxyphenyl)-ethyl)-amino]-5-methyl-4-(3′,4′,5′-trimethoxyphenyl)-hexane and of its salts, wherein the acid salt of the compound with an optically active form of O,O′-dibenzoyl-tartaric acid is prepared, as a diastereomer mixture, by neutralizing the free base or by double decomposition of another salt of the compound, and the mixture is separated by crystallization, and the pure diastereomers thus obtained are converted in a conventional manner to the free bases of the enantiomeric forms of 4-cyano-1-[N-methyl-N-(2′-(3″,4″-dimethoxyphenyl)-ethyl)-amino]-5-methyl-4-(3′,4′,5′-trimethoxyphenyl)-hexane or to salts thereof.

2. A process as claimed in claim 1, wherein the acid O,O′-dibenzoyltartrate is prepared, as a diastereomer mixture, using isopropanol as the solvent.

3. A process as claimed in claims 1 and 2, wherein (+)-O,O′-dibenzoyl-D-tartaric acid, its hydrate or one of its mono-alkali metal salts is used.

**Revendications**

1. Procédé pour l'obtention des formes énantiomères du cyano-4 [N-méthyl N-(diméthoxy-3″,4″ phényl) éthyl-2′) amino]-1 méthyl-5 (triméthoxy-3′,4′,5′ phényl)-4 hexane et de ses sels, caractérisé en ce que l'on prépare, par neutralisation de la base libre ou par double réaction d'un sel de ce composé, un sel acide de cette base et d'une forme optiquement active de l'acide O,O′-dibenzoyl-tartrique à l'état d'un mélange des diastéréo-isomères, que l'on sépare par cristallisation, les diastéréo purs ainsi obtenus étant ensuite transformés dans les bases libres des formes énantiomères du cyano-4 [N-méthyl N-(diméthoxy-3″,4″ phényl) éthyl-2′) amino]-1 méthyl-5 (triméthoxy-3′,4′,5′ phényl)-4 hexane ou en leurs sels.

2. Procédé suivant la revendication 1, caractérisé en ce que le tartrate de O,O′-dibenzoyle acide est préparé à l'état de mélange de diastéréo-isomères en employant comme solvant l'isopropanol.

3. Procédé suivant les revendications (+)-O,O′-dibenzoyl-D-tartrique, son hydrate ou un de ses sels mono-alcalins.